# EUROPÄISCHE PATENTSCHRIFT

(11) **EP 3 213 794 B1**
(45) Veröffentlichungstag und Bekanntmachung des Hinweises auf die Patenterteilung: **29.07.2020**
(21) Anmeldenummer: 17000341.2
(22) Anmeldetag: 03.03.2017
(51) Int. Cl.: A61N 2/02, A61N 1/40

(54) **BEFELDUNGSGERÄT ZUR ERZEUGUNG ELEKTROMAGNETISCHER WELLENZÜGE**
IRRADIATION DEVICE FOR GENERATING ELECTROMAGNETIC WAVE TRAINS
APPAREIL D'ÉMISSION DE CHAMP DESTINÉ À PRODUIRE DES TRAINS D'ONDES ÉLECTROMAGNÉTIQUES

(30) Priorität: 03.03.2016 DE 102016002482
(43) Veröffentlichungstag der Anmeldung: 06.09.2017
(73) Patentinhaber: Pries, Wolfgang, 86825 Bad Wörishofen (DE)
(72) Erfinder: Pries, Wolfgang, 86825 Bad Wörishofen (DE)
(74) Vertreter: Liebl, Thomas

(56) Entgegenhaltungen:
- DE-A1-102008 037 979
- DE-A1-102014 001 433
- ALEXANDRA GRAMOWSKI-VOSS ET AL: "Enhancement of Cortical Network Activity in vitro and Promotion of GABAergic Neurogenesis by Stimulation with an Electromagnetic Field with a 150?MHz Carrier Wave Pulsed with an Alternating 10 and 16?Hz Modulation", FRONTIERS IN NEUROLOGY, Bd. 6, 14. Juli 2015 (2015-07-14), XP055384201, ISSN: 1664-2295, DOI: 10.3389/fneur.2015.00158

## Beschreibung

Die Erfindung betrifft ein Befeldungsgerät zur Erzeugung elektromagnetischer Wellenzüge nach dem Oberbegriff des Anspruchs 1.

Ein bekanntes, gattungsgemäßes Befeldungsgerät zur Erzeugung elektromagnetischer Wellenzüge (Alexandra Gramowski-Voss et al: "Enhancement of cortical network activity in vitro and promotion of GABAergic neurogenesis by stimulation with an electromagnetic field with a 150 MHz carrier wave pulsed with an alternating 10 and 16 Hz modulation" frontiers in Neurology, Bd. 6, 14. Juli 2015 (2015-07-14), XP055384201, ISSN: 1664-2295, DOI: 10.3389/fneur.2015.00158), weist eine Elektronik-Geräteeinheit mit einer Hochfrequenzoszillationsstufe zur Erzeugung eines Hochfrequenzwellenzugs (HF-Wellenzug) mit einer Frequenz von 150 MHz, und eine durch Kabel mit der Elektronik-Geräteeinheit verbundenen Befeldungskapsel auf, in der eine Magnetspule als Befeldungsspule in der Art einer magnetischen Antenne enthalten ist. Für eine getaktete Unterbrechung des HF-Wellenzugs ist ein Niederfrequenztaktgeber (NF-Taktgeber) vorgesehen zur Erzeugung einer Taktfrequenz mit zugeordneter Periodendauer und eine damit gekoppelte Unterbrechereinheit, die den HF-Wellenzug jeweils für eine halbe Periodendauer unterbricht, dergestalt dass im Niederfrequenz-Takt 150MHz-HF-Wellenzugpakete mit halber Periodendauer und anschließender Pausenzeit von einer halben Periodendauer erzeugt werden. Die Taktfrequenz beträgt hier 10 Hz oder 16 Hz und die Abstrahlung erfolgt mit maximal 0,125 pT (pico Tesla). Der Befeldungsabstand ist hier fest auf 83 mm eingestellt, wobei in einer "in vitro"-Anwendung leere "multiwell plates" als Abstandshalter vorgeschlagen sind. Wie diese Abstandshalter angeordnet sein sollen, um eine Befeldungskapsel auf dem vorgegebenen Abstand zu halten, ist nicht dargestellt.

Bei einem weiter bekannten, ähnlichen Befeldungsgerät (DE 10 2014 001 433 A1) liegt in einer "in vitro"-Anwendung eine Befeldungskapsel auf einer Abstützfläche und darüberliegend können in unterschiedlichen Abständen neuronale Vorläuferzellen befeldet werden, um unter anderem den Einfluss unterschiedlicher Abstände auf das Befeldungsergebnis zu untersuchen. In einer konkret dargestellten Anordnung wird dies dadurch erreicht, dass mehrere Befeldungsgruppen übereinandergestapelt auf der Befeldungskaspsel angeordnet sind.

Bei einem bekannten Befeldungsgerät (DE 10 2008 037 979 A1) enthält eine Elektronik-Geräteeinheit mit Energieversorgung eine Hochfrequenzoszillatorstufe zur Erzeugung eines Hochfrequenzwellenzugs (HF-Wellenzug) mit einer einstellbaren Frequenz, die zum Beispiel 150 MHz sein kann. Zudem sind an die Elektronik-Geräteeinheit zwei Befeldungskapseln angeschlossen, in der jeweils eine Magnetspule als Befeldungsspule in der Art einer magnetischen Antenne enthalten ist. Diese Befeldungskapseln werden zur Befeldung von Körperteilen von Lebewesen direkt auf einem Körperteil aufgesetzt und angelegt, insbesondere bei einer Anwendung des Befeldungsgeräts zur Arthrose-Therapie und zur transkraniellen Stimulation, wobei der Hochfrequenzwellenzug mit einer Niederfrequenz von 10,0 Hz oder 10,2 Hz amplitudenmoduliert wird. Die zwei Befeldungskapseln sind mit ihren aktiven Seiten aufeinander zugerichtet an einer Halterung mit zwei Bügelteilen befestigt, die über eine einstellbare Schiebeführung zur Fixierung der Befeldungskapseln miteinander verbunden sind. Die Befeldungskapseln werden mit der Halterung auf ein zu befeldendes Körperteil, beispielsweise ein Kniegelenk aufgesetzt und dort durch Zusammenschieben der Schiebeführung fixiert.

Aufgabe der Erfindung ist es, ein Befeldungsgerät zur Verfügung zu stellen, das sowohl als Laborgerät als auch als Therapiegerät für im Vergleich zum Stand der Technik andere weitere Befeldungseinsätze, insbesondere für Untersuchungen an kultivierten Hautstrukturen und zur Therapie von geschädigten Hauptbereichen bei einfacher Handhabung wirkungsvoll einsetzbar ist.

Diese Aufgabe wird mit den Merkmalen des Anspruchs 1 gelöst.

Gemäß Anspruch 1 ist in der Elektronik-Geräteeinheit zusätzlich zur Hochfrequenzoszillatorstufe für eine getaktete Unterbrechung des HF-Wellenzugs ein Niederfrequenztaktgeber (NF-Taktgeber) vorgesehen zur Erzeugung einer Taktfrequenz von 15 Hz mit zugeordneter Periodendauer (66,6 ms). Mit dem NF-Taktgeber ist eine Unterbrechereinheit gekoppelt, die den HF-Wellenzug jeweils für eine halbe Periodendauer unterbricht dergestalt dass im 15 Hz-Takt 150MHz-HF-Wellenzugpakete mit halber Periodendauer und anschließender Pausenzeit von einer halben Periodendauer erzeugt werden, um einer biologischen Resonanz gerecht zu werden.

Diese erzeugten 150MHz-HF-Wellenzugpakete werden der wenigstens einen Befeldungskapsel zur Abstrahlung mit einer Induktionsstärke von 0,5 pT (pico Tesla) zugeführt. Dazu ist die wenigstens eine Befeldungskapsel jeweils auf einer Abstandspositioniervorrichtung montiert, mit der sie mit ihrer aktiven Abstrahlfläche in einem vorgebbaren definierten Abstand zu einem Befeldungsflächenbereich eines Befeldungsobjekts positionierbar ist. Mit einer solchen Abstandspositioniervorrichtung kann eine Befeldungskapsel auch dort verwendet werden, wo sie nicht direkt aufgesetzt und anlegbar ist, wie beispielsweise auf einem durch Verletzung geschädigten offenen Hautbereich eines Lebewesens oder bei einer Zellkulturschale mit kultivierten Hautstrukturen.Die Abstandspositioniervorrichtung ist dazu als Gestell mit längeneinstellbaren Abstandshaltern ausgeführt sein, wobei dann die Abstandshalter vorzugweise in einem Bereich um einen Befeldungsflächenbereich aufsetzbar und abstützbar sind. Mit den einstellbaren Abstandshaltern kann einfach, bequem und genau der Relativabstand zwischen der aktiven Abstrahlfläche der Befeldungskapsel und dem Befeldungsflächenbereich eingestellt und variiert werden, wodurch entsprechend auch die Induktionsstärke im Befeldungsflächenbereich und damit die Stärke der Beeinflussung variierbar ist. Als weitere variierbare Befeldungsparameter zur Erzielung einer biologischen Resonanz stehen die Befeldungszeitdauer, die Anzahl von Befeldungen und die dazwischen einzuhaltenden Pausezeiten zur Verfügung.

Das erfindungsgemäße Befeldungsgerät ist besonders geeignet für eine "in vivo"-Anwendung zur Befeldung von durch Verletzungen und/oder Krankheiten geschädigten offenen Hauptbereichen eines Lebewesens. Es hat sich gezeigt, dass mit mehreren in zeitlichen Abständen aufeinanderfolgenden Befeldungen eine Heilung positiv und reproduzierbar beeinflussbar ist und insbesondere ein Wundverschluss beschleunigt herbeigeführt werden kann. Beispielsweise wurde ein offenes Bein mit Ulcus cruris im Unterschenkelbereich innerhalb von zwei Behandlungswochen insgesamt siebenmal befeldet; nach ca. 10 Monaten war der offene Hautbereich geheilt.

Mit dem Befeldungsgerät können vorteilhaft in einer "in vitro"-Anwendung kultivierte Hautstrukturen auf einer Trägermembran in einer Zellkulturschale, insbesondere Keratinozyten-Zellkulturen im Labor im Bereich einer biologischen Toleranz befeldet werden. Die Wirkung der Befeldung auf die Zellkulturen kann dabei labormäßig gezielt und in Reihenversuchen, beispielsweise mit variierenden Befeldungszeiten und variierenden Pausenzeiten durch Messungen ausgewertet werden. Es wurden bereits positive reproduzierbare Wirkungen bei Heilprozessen und Alterungsprozessen an den kultivierten Hautstrukturen festgestellt.

Die sehr kleine Induktionsstärke von 0,5 pT (pico Tesla) an der Befeldungskapsel wurde einerseits gewählt, um Befeldungsschäden an Hautstrukturen, insbesondere Verbrennungen bei jeglicher Anwendung sicher auszuschließen und um andererseits Störbeeinflussungen der Umgebung durch die getakteten HF-Wellenzugpakete zu vermeiden. Ein bevorzugter, gut geeigneter Relativabstand zwischen der aktiven Abstrahlfläche der Befeldungskapsel und dem Befeldungsflächenbereich liegt zwischen 4 mm und 25 mm, vorzugsweise bei 6 - 10 mm. Je nach dem eingestellten Relativabstand ist die Induktionsstärke der Befeldungskapsel am Befeldungsflächenbereich, insbesondere einem Hautbereich eines Lebewesens oder einer kultivierten Halbstruktur, entsprechend reduziert.

Für die vorstehenden Verwendungen ist es für eine einfache Handhabung zweckmäßig, eine einzige Befeldungskapsel auf einer Abstandspositioniervorrichtung anzuordnen und dort für einen gegebenenfalls erforderlichen Austausch einfach lösbar zu montieren.

Anhand einer Zeichnung wird die Erfindung weiter erläutert.

Es zeigen:
- Fig. 1: ein Befeldungsgerät in der Anwendung zur Befeldung kultivierter Hautstrukturen in einer Seitenansicht,
- Fig. 2: das Befeldungsgerät nach Fig. 1 in einer Draufsicht,
- Fig. 3: das Befeldungsgerät nach Fig. 1 (ohne Elektronik-Geräteeinheit) in einer Anwendung zur Befeldung eines geschädigten Hautbereichs an einem Bein in einer Seitenansicht,
- Fig. 4: die Darstellung nach Fig. 3 in einer Draufsicht, und
- Fig. 5: eine Darstellung des getakteten HF-Wellenzugs, der bei der Befeldung verwendet wird.

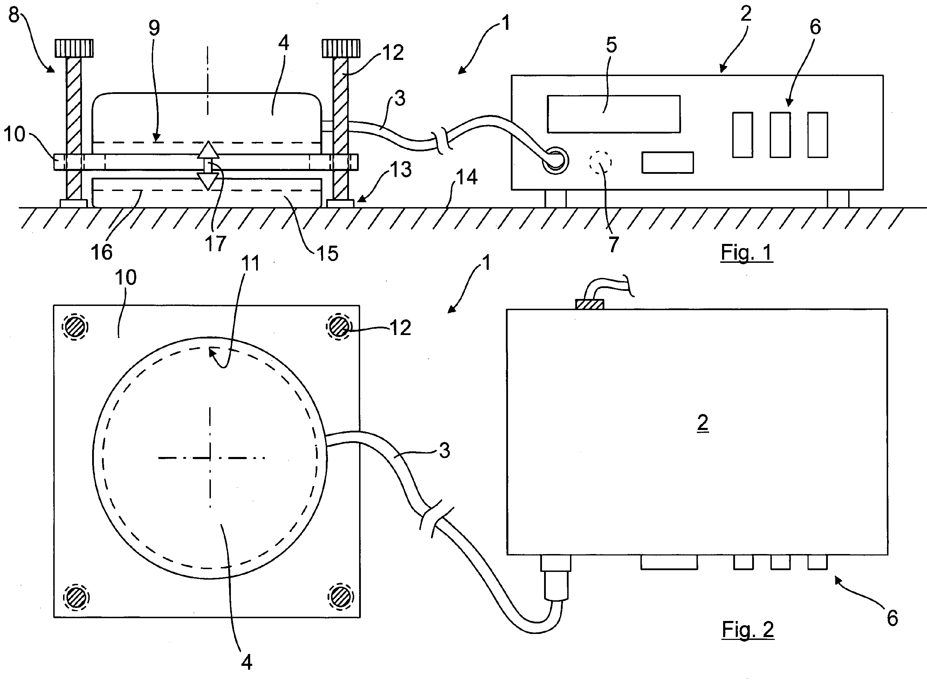

In Fig. 1 ist ein Befeldungsgerät 1 in einer Seitenansicht dargestellt mit einer Elektronik-Geräteeinheit 2, an die mit einem Kabel 3 eine Befeldungskapsel 4 angeschlossen ist. Die Elektronik-Geräteeinheit 2 weist beispielhaft ein Display zur Anzeige der eingestellten/abgelaufenen Befeldungszeit, der Induktionsstärke, etc. auf. Weiter sind diverse Schalter und Stellelemente 6 vorgesehen, insbesondere zum Einschalten einer Versorgungsspannung, zu Zeiteinstellungen, etc. Weiter ist strichliert eine mögliche Anschlussstelle 7 für eine weitere Befeldungskapsel 4 angegeben.

Die Befeldungskapsel 4 ist auf einer Abstandspositioniervorrichtung 8 mit ihrer aktiven Abstrahlfläche 9 nach unten weisend angebracht. Die Abstandspositioniervorrichtung 8 besteht aus einer Montageplatte 10 mit einem kreisförmigen Ausschnitt 11 unter der aktiven Abstrahlfläche 9 der Befeldungskapsel 4, dergestalt, dass diese mit einem umlaufenden Randbereich auf der Montageplatte 10 aufliegt und dort fixierbar ist. In den Eckbereichen der Halteplatte 10 sind Gewindebohrungen eingebracht, in denen jeweils Abstandshalter 12 als Gewindebolzen aufgenommen sind. Die Abstandshalter 12 stehen mit ihrem unteren Ende 13 jeweils auf der Tischplatte 14 eines Labortisches.

Unter der Befeldungskapsel 4 bzw. unter der Montageplatte 10 ist für ein Befeldungsexperiment eine Zellkulturschale 15 aufgestellt. Diese enthält eine Trägermembran 16 mit kultivierten Hautstrukturen. Mit der Gewindestellung der Abstandshalter 12 kann der Relativabstand zwischen der aktiven Abstrahlfläche 9 der Befeldungskapsel 4 und der Trägermembran 16 bzw. der dortigen kultivierten Hautstrukturen eingestellt werden und ist hier entsprechend dem Doppelpfeil 17 auf 18 mm eingestellt.

Aus der Draufsicht der Fig. 2 ist zudem die kreisrunde Form der Befeldungskapsel und die hier beispielhaft gewählte quadratische Form der Montageplatte 10 ersichtlich. Grundsätzlich kann eine im Abstand einstellbare Halterung zur Abstützung der Befeldungskapsel 4 gegenüber der Tischplatte 14 auch mit anderen geeigneten Mitteln ausgeführt sein.

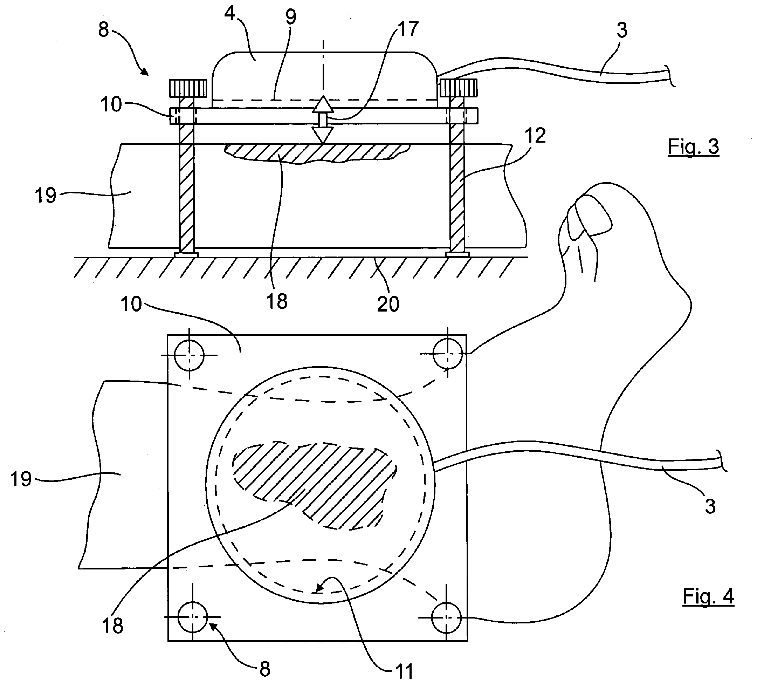

In den Fig. 3 und 4 ist das Befeldungsgerät 1 (die Elektronik-Geräteeinheit 2 entspricht den Fig. 1 und 2, wurde der Übersichtlichkeit halber aber weggelassen) in einer "in vivo"-Anwendung zur Befeldung eines geschädigten offenen Hautbereichs nämlich eines Ulcus cruris am distalen Unterschenkel eines menschlichen Beins 19 dargestellt. Das Bein 19 liegt dabei auf einer Unterlage 20, auf der sich die Abstandspositioniervorrichtung 8 mit weit aus der Montageplatte 10 herausgedrehten Abstandshaltern 12 so abstützt, dass der Relativabstand (Doppelpfeil 17) zwischen der aktiven Abstrahlfläche 9 der Befeldungskapsel 4 und dem Hautbereich 18 ca. 6 - 10 mm beträgt, wobei die Befeldungskapsel 4 oberhalb des Hautbereichs 18 positioniert ist.

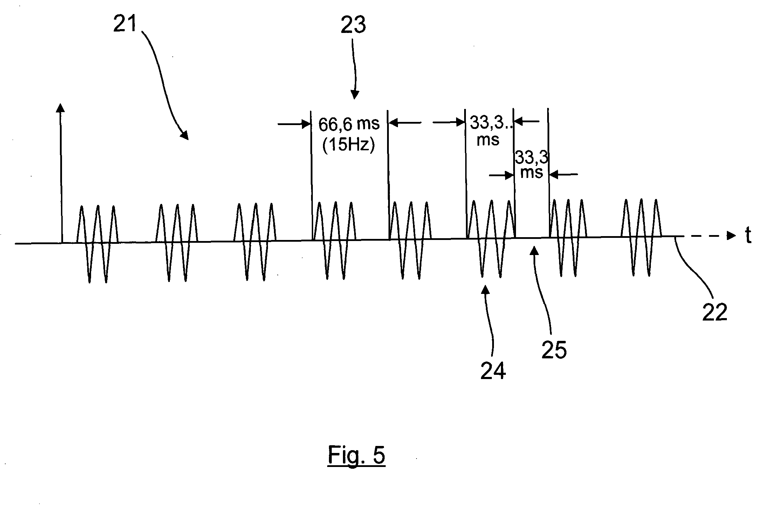

In Fig. 5 ist der für die Befeldung eingesetzte getaktete Wellenzug 21 auf einer Zeitachse 22 dargestellt:
Der in der Elektronik-Geräteeinheit 2 mit einer Hochfrequenzoszillatorstufe erzeugte Hochfrequenzwellenzug (HF-Wellenzug) wird zum Aufbau einer biologischen Resonanz getaktet regelmäßig unterbrochen. Dazu ist ein Niederfrequenztaktgeber (NF-Taktgeber) vorgesehen, welcher eine Taktfrequenz von 15 Hz erzeugt. Diese entspricht einer Periodendauer 23 von 66,6 ms. Mit dem Niederfrequenztaktgeber ist eine Unterbrechereinheit gekoppelt, welche den HF-Wellenzug jeweils für eine halbe Periodendauer unterbricht dergestalt, dass jeweils im 15 Hz-Takt ein 150MHz-HF-Wellenzugpaket 24 mit der halben Periodendauer von 33,3 ms und einer anschließenden Pausenzeit 55 mit ebenfalls einer Periodendauer von 33,3 ms erzeugt wird.

Der 150MHz-HF-Wellenzug ist somit in einer Art von Puls-Pausen moduliert, wobei die Pulse aus den Wellenzugpaketen bestehen (ersichtlich sind die Anzahl der HF-Wellen der Wellenzugpakte 24 der Übersichtlichkeit halber nicht maßstabsgetreu mit zu wenig Wellen dargestellt). Die Befeldung von Hautbereichen von Lebewesen ebenso wie von kultivierten Hautstrukturen mit dem dargestellten getakteten Wellenzug 21 führt zu einer biologischen Resonanz und zeigt deutlich erkennbare und messbare Beeinflussungen mit zum Teil positiv verwertbaren und reproduzierbaren Wirkungen.

## Patentansprüche

1. Befeldungsgerät zur Erzeugung elektromagnetischer Wellenzüge,
mit einer Elektronik-Geräteeinheit (2) mit einer Hochfrequenzoszillationsstufe zur Erzeugung eines Hochfrequenzwellenzugs (HF-Wellenzug) mit einer Frequenz von 150 MHz, und
mit wenigstens einer durch Kabel (3) mit der Elektronik-Geräteeinheit (2) verbundenen Befeldungskapsel (4), in der eine Magnetspule als Befeldungsspule in der Art einer magnetischen Antenne enthalten ist, wobei zur getakteten Unterbrechung des HF-Wellenzugs ein Niederfrequenztaktgeber (NF-Taktgeber) zur Erzeugung einer Taktfrequenz mit zugeordneter Periodendauer und eine damit gekoppelte Unterbrechereinheit vorgesehen sind, die den HF-Wellenzug jeweils für eine halbe Periodendauer unterbricht, dergestalt
dass im Niederfrequenz-Takt 150MHz-HF-Wellenzugpakete (24) mit halber Periodendauer und anschließender Pausenzeit (25) von einer halben Periodendauer erzeugt werden,
**dadurch gekennzeichnet,**
**dass** der Niederfrequenztaktgeber eine Taktfrequenz von 15 Hz mit zugeordneter Periodendauer von 66,6 ms erzeugt,
**dass** die erzeugten 150MHz-HF-Wellenzugpakete (24) der wenigstens einen Befeldungskapsel (4) zur Abstrahlung mit einer Induktionsstärke von 0,5 pT (pico Tesla) zugeführt werden,
**dass** die wenigstens eine Befeldungskapsel (4) jeweils auf einer Abstandspositioniervorrichtung (8) montiert ist, mit der die wenigstens eine Befeldungskapsel (4) mit ihrer aktiven Abstrahlfläche (9) in einem vorgebbaren definierten Abstand (17) zu einem Befeldungsflächenbereich (16; 18) eines Befeldungsobjekts positionierbar ist, und
**dass** die Abstandspositioniervorrichtung (8) ein Gestell mit längseinstellbaren Abstandshaltern (12) ist, wobei die Abstandshalter (12) im Bereich um einen Befeldungsflächenbereich (16; 18) aufsetzbar und abstützbar sind.

2. Befeldungsgerät nach Anspruch 1, **dadurch gekennzeichnet, dass** der Befeldungsflächenbereich in einer "in vivo"-Anwendung des Befeldungsgeräts (1) ein durch Verletzung und/oder Krankheit geschädigter offener Hautbereich (18) eines Lebewesens ist, der mit mehreren in zeitlichen Abständen aufeinanderfolgenden Befeldungen für einen Wundverschluss beeinflussbar ist.

3. Befeldungsgerät nach Anspruch 1, **dadurch gekennzeichnet, dass** der Befeldungsflächenbereich in einer "in vitro"-Anwendung des Befeldungsgeräts (1) eine Trägermembran (16) einer Zellkulturschale (15) mit kultivierten Hautstrukturen, insbesondere mit Keratinozyten-Zellkulturen ist.

4. Befeldungsgerät nach einem der Ansprüche 1 bis 3, **dadurch gekennzeichnet, dass** der mit der Abstandspositioniervorrichtung (8) einstellbare Relativabstand (17) zwischen der aktiven Abstrahlfläche (9) der Befeldungskapsel (4) und dem Befeldungsflächenbereich (16; 18) zwischen 4 mm und 25 mm, vorzugsweise zwischen 6 mm und 10 mm beträgt.

5. Befeldungsgerät nach einem der Ansprüche 1 bis 4, **dadurch gekennzeichnet, dass** eine Befeldungskapsel (4) auf einer Abstandspositioniervorrichtung (8) lösbar montiert ist.

## Claims

1. Irradiation device for generating electromagnetic wave trains, comprising an electronics device unit (2) with a radiofrequency oscillator stage for generating a radiofrequency wave train (RF wave train) with a frequency of 150 MHz, and
comprising at least one irradiation capsule (4) which is connected by means of a wire (3) to the electronics device unit (2) and in which a magnetic coil is contained as an irradiation coil in the style of a magnetic antenna, wherein a low-frequency clock generator (LF clock generator) for generating a clock frequency with an associated period and an interrupter unit coupled therewith are provided for the clocked interruption of the RF wave train, said interrupter unit interrupting the RF wave train for half a period in each case, in such a way
that 150 MHz RF wave train packets (24) with half a period and a subsequent pause time (25) of half a period are generated with the low-frequency clock,
**characterized**
**in that** the low-frequency clock generator generates a clock frequency of 15 Hz with an associated period of 66.6 ms,
**in that** the generated 150 MHz RF wave train packets (24) are supplied to the at least one irradiation capsule (4) for emission with a magnetic induction strength of 0.5 pT (picotesla),
**in that** each one of the at least one irradiation capsule (4) is mounted on a distance-positioning apparatus (8), by means of which the active emission surface (9) of the at least one irradiation capsule (4) is positionable at a predeterminable defined distance (17) from a surface region to be irradiated (16; 18) of an object to be irradiated, and
**in that** the distance-positioning apparatus (8) is a frame with longitudinally adjustable spacers (12), wherein the spacers (12) are mountable and supportable in the region around a surface region to be irradiated (16; 18).

2. Irradiation device according to Claim 1, **characterized in that** the surface region to be irradiated in an "in vivo" application of the irradiation device (1) is an open skin region (18), damaged by injury and/or disease, of a living being, said skin region being influenceable in respect of sealing the wound by way of a plurality of instances of irradiation that are successive in time.

3. Irradiation device according to Claim 1, **characterized in that** the surface region to be irradiated in an "in vitro" application of the irradiation device (1) is a carrier membrane (16) of a Petri dish (15) with cultivated skin structures, in particular with keratinocyte cell cultures.

4. Irradiation device according to any one of Claims 1 to 3, **characterized in that** the relative distance (17) between the active emission surface (9) of the irradiation capsule (4) and the surface region to be irradiated (16; 18), adjustable by means of the distance-positioning apparatus (8), ranges between 4 mm and 25 mm, preferably between 6 mm and 10 mm.

5. Irradiation device according to any one of Claims 1 to 4, **characterized in that** an irradiation capsule (4) is detachably assembled on a distance-positioning apparatus (8).

## Revendications

1. Appareil d'émission de champ destiné à produire des trains d'ondes électromagnétiques, comprenant
une unité d'appareil électronique (2) munie d'un étage d'oscillation haute fréquence destiné à produire un train d'ondes haute fréquence (train d'ondes HF) d'une fréquence de 150 MHz, et
au moins un boîtier d'émission de champ (4) relié à l'unité d'appareil électronique (2) par un câble (3) et contenant une bobine magnétique en tant que bobine d'émission de champ à la manière d'une antenne magnétique,
dans lequel, pour une interruption cadencée du train d'ondes HF, un générateur de cadence basse fréquence (cadence NF), destiné à produire une cadence d'une durée de période associée, et une unité d'interruption couplée à celui-ci et qui interrompt le train d'ondes HF respectivement pendant la durée d'une demi-période sont prévus, de telle sorte
qu'à la cadence NF des paquets de train d'ondes HF de 150 MHz (24) sont produits avec la durée d'une demi-période et un temps de pause consécutif (25) de la durée d'une demi-période,
**caractérisé en ce que**
le générateur de cadence basse fréquence produit une cadence de 15 Hz d'une durée de période associée de 66,6 ms,
les paquets de train d'ondes HF de 150 MHz (24) produits sont amenés audit au moins un boîtier d'émission de champ (4) en vue d'une émission d'une intensité d'induction de 0,5 pT (pico Tesla),
ledit au moins un boîtier d'émission de champ (4) est monté respectivement sur un dispositif de positionnement espacé (8) qui permet de positionner ledit au moins un boîtier d'émission de champ (4) par sa surface d'émission active (9) à une distance définie prédéfinissable (17) par rapport à une zone de surface d'émission de champ (16 ; 18) d'un objet d'émission de champ, et
le dispositif de positionnement espacé (8) est un bâti muni d'entretoises (12) réglables en longueur, les entretoises (12) pouvant être posées ou supportées dans la zone autour d'une zone de surface d'émission de champ (16 ; 18).

2. Appareil d'émission de champ selon la revendication 1, **caractérisé en ce que** la surface d'émission de champ dans une application in vivo de l'appareil d'émission de champ (1) est une zone cutanée (18) ouverte, endommagée par une lésion et/ou une maladie, d'un être vivant qui peut être affectée par plusieurs émissions de champ successives à intervalles dans le temps en vue de fermer une plaie.

3. Appareil d'émission de champ selon la revendication 1, **caractérisé en ce que** la zone de surface d'émission de champ dans une application in vitro de l'appareil d'émission de champ (1) est une membrane de support (16) d'une coupelle de culture cellulaire (15) contenant des structures cutanées cultivées, en particulier des cultures cellulaires de kératinocytes.

4. Appareil d'émission de champ selon l'une quelconque des revendications 1 à 3, **caractérisé en ce que** la distance relative (17) réglable à l'aide du dispositif de positionnement espacé (8) entre la surface d'émission active (9) du boîtier d'émission de champ (4) et la zone de surface d'émission de champ (16 ; 18) est comprise entre 4 mm et 25 mm, de préférence entre 6 mm et 10 mm.

5. Appareil d'émission de champ selon l'une quelconque des revendications 1 à 4, **caractérisé en ce qu'**un boîtier d'émission de champ (4) est monté de manière amovible sur un dispositif de positionnement espacé (8).
